# EUROPEAN PATENT APPLICATION

(11) **EP 2 572 634 A1**
(43) Date of publication of application: **27.03.2013**
(21) Application number: 11182512.1
(22) Date of filing: 23.09.2011
(51) Int. Cl.: A61B 5/04, A61B 5/0402, A61B 5/0452, A61B 5/046, A61B 5/0464

(54) **A system and computer program product for automatically distinguishing atrial flutter from atrial fibrillation**

(71) Applicant: Ruprecht-Karls-Universität Heidelberg, 69117 Heidelberg (DE)
(72) Inventor: Scholz, Eberhard, 69120 Heidelberg (DE); Sager, Sebastian, 69117 Heidelberg (DE); Katus, Hugo, 69120 Heidelberg (DE)
(74) Representative: Lucke, Andreas

(57) **Abstract**

The present invention relates to a system for automatically distinguishing atrial flutter from atrial fibrillation based on a sequence of R-R-intervals of ECG data. The system of the invention comprises means adapted to calculate or simulate an R-R-pattern from a model which is based on a periodic or almost periodic signal representing peaks of an atrial flutter signal, a first AV-block scheme blocking respective ones of the atrial excitations while transmitting the others with a predetermined transmittal delay and at least one more AV-block scheme blocking respective ones of the excitations transmitted by the previous AV-block scheme and transmitting the others with a predetermined transmittal delay. The excitations transmitted by the last AV-block scheme constitute the calculated R-R-pattern. The system further comprises consistency checking means adapted to automatically check whether said sequence of R-R-intervals of the ECG-data is to some predetermined degree consistent with said calculated or simulated R-R-pattern. The system is configured to identify the ECG-data to be based on atrial flutter if said predetermined degree of consistency is found.

## Description

### FIELD OF THE INVENTION

The present invention is in the field of medical technology. In particular, the present invention relates to a system and a computer program product for automatically distinguishing atrial flutter from atrial fibrillation.

### BACKGROUND OF THE INVENTION

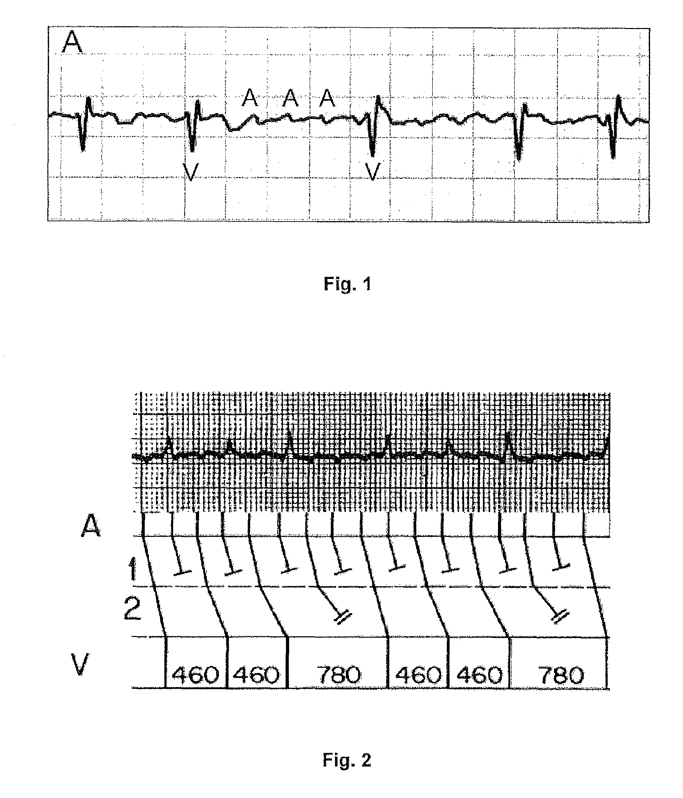

Atrial fibrillation is the most common cardiac arrhythmia and leads to both, an increased mortality and an increased risk of stroke. Atrial fibrillation is characterized by a fibrillation or quivering of the heart muscles of the atrial chambers instead of a coordinated contraction. The irregular stimulation of the atrial chambers is electrically transmitted to the ventricular chambers via the atrioventricular (AV) node. The electrical stimulation as transmitted by the AV-node has a lower frequency than the fibrillation and is typically entirely arrhythmic, the so-called "arrhythmia absoluta". An example of an ECG of a patient with atrial fibrillation is shown in Fig. 1. The irregular high frequency fibrillation of the atrial chambers is designated by "A" in Fig. 1, while the ventricular chambers are stimulated arrhythmically, as is indicated by the letter "V". Accordingly, such arrhythmic beats of the ventricular chambers in combination with hardly discernible high-frequency atrial excitations is quasi pathognomonic for atrial fibrillation.

In recent years, an invasive therapy has been developed that allows treating atrial fibrillation. The cornerstone of this method is known as "pulmonary vein isolation" (PVI), in which the four pulmonary veins leading to the left atrial chamber are electrically isolated from the tissue of the atrium by obliteration using an ablation catheter. This procedure is based on the observation that ectopic beats arising from muscular sleeves located in the ends of the pulmonary veins play a crucial role in the generation and maintaining of atrial fibrillation, see e. g. Haïssaguerre M, Jaïs P, Shah DC, Takahashi A, Hocini M, Quniou G, Garrigue S, Le Mouroux A, Le Métayer P, Clémenty J. Spontaneous initiation of atrial fibrillation by ectopic beats originating in the pulmonay veins. N Engl J Med. 1998; 339;659-666.

Due to its curative approach in combination with the high prevalence of atrial fibrillation, the number of performed PVIs is growing rapidly. However, it is well recognized that this treatment may be associated with severe side effects and complications including secondary periodic atrial tachycardia, which is also referred to as "atrial flutter" herein, see e. g. Veenhuyzen GD, Knecht S, O'Neill MD, Phil D, Wright M, Nault I, Weerasooriya R, Miyazaki S, Sacher F, Hocini M, Jaïs P, Haïssaguerre M. Atrial tachycardias encountered during and after catheter ablation for atrial fibrillation: part I: classification, incidence, management. Pacing Clin Electrophysiol. 2009; 32:393-398.

The reasons for this type of tachycardia are either rapid focal depolarizations or reentrant circuits. From the patient's point of view, atrial flutter may feel similar or even worse than atrial fibrillation and in the long turn may severely worsen the cardiac pump function. Therapy of choice usually consists in a second invasive procedure, in which the aforementioned mechanism is analyzed and the tachycardia is terminated by targeted ablation. However, this is of course only true if the flutter is reliably diagnosed and in particular, if it is correctly distinguished from a recurrence of atrial fibrillation. This is especially important as due to the increasing number of PVIs, large numbers of secondary tachycardia are to be expected.

### SUMMARY OF THE INVENTION

The problem underlying the invention is to provide means assisting the cardiologist to reliably distinguish atrial flutter from atrial fibrillation. In standard cases, an experienced cardiologist can easily distinguish atrial fibrillation from atrial flutter. The main difference between atrial fibrillation and common forms of atrial flutter is that whereas atrial excitation causes irregular high-frequency waves in atrial fibrillation, typical periodic flutter waves can be observed in most cases of atrial flutter. Furthermore, atrial fibrillation generally leads to irregular, non-periodic R-R-patterns in the ECG, the so-called "arrhythmia absoluta". Thus, hardly discernible irregular atrial activation in combination with an irregular ventricular response is quasi pathognomonic for atrial fibrillation. However, two electrophysiological phenomena can severely complicate this differentiation: (1) In contrast to common forms of atrial flutter, where atrial activation is easily visible as a so-called "sawtooth pattern", atrial activation may be vanishingly small in atrial flutter secondary to PVI. (2) Due to complex atrio-ventricular conduction patterns, an irregular ventricular response may result, mimicking an "arrhythmia absoluta".

The underlying mechanism of this irregular atrio-ventricular conduction is as follows. It is known that the AV-node may block some of the atrial excitations or P-waves instead of transmitting them to the ventricular chamber. In the art, two different types of AV-blocking schemes are generally known, one as the Mobitz AV-block scheme and the other as the Wenckebach AV-black scheme. Due to the blocking of atrial excitations, the regular pattern of the atrial flutter is disrupted. Nevertheless, an experienced cardiologist would generally be able to recognize these types of AV-block schemes and will hence not confuse atrial flutter with atrial fibrillation.

However, what is not so much known in the art is that the AV-node may display a so-called "multilevel" AV-black. A multi-level AV-black may be thought of as a sequence or hierarchy of two or even more AV-blacking schemes carried out in sequence. This phenomenon has been reported in a work by Kosowsky et al. in the 1970s, Kosowsky BD, Latif P, Radaff AM. Multilevel atrioventricular block. Circulation. 1976; 54:914-921. However, to the knowledge of the inventors, no practical use in clinical diagnosis has been made of the multi-level AV-block scheme.

It turns out that if two different AV-blocks are combined, the resulting R-R-pattern may already be rather complex and appear highly irregular. Consequently, it is very likely that in case of a multi-level AV-block, even an experienced cardiologist will no longer be able to recognize atrial flutter. Instead, there is a high risk that due to the irregularity of the R-R-pattern, the cardiologist will attribute this incorrectly to "arrhythmia absoluta" caused by atrial fibrillation. This comes especially true for PVI related atrial flutter where atrial excitations are often vanishingly small and therefore do not help making the right diagnosis. However, a wrong diagnosis may of course have fatal consequences for the patient. For example, as mentioned before, atrial flutter is often highly symptomatic and may result in a severely reduced pump function of the heart. If the patient should have a two or more level AV-block, for the reasons mentioned above, there is a considerable risk that the cardiologist will misdi-agnose atrial flutter as recurrence af atrial fibrillation.

In order to rule out these types of errors, the present invention provides a system for automatically distinguishing atrial flutter from atrial fibrillation purely based on a sequence of R-R-intervals of ECG-data as defined in claim 1. Preferable embodiments are defined in the dependent claims.

According to the invention, the system comprises means adapted to calculate an R-R-pattern from a model which is based on
- a periodic or almost periodic signal representing peaks of an atrial flutter signal,
- a first AV-block scheme blocking respective ones of the excitations corresponding to said peaks of the atrial flutter signal while transmitting the others with a predetermined transmittal delay,
- at least one more AV-block scheme blocking respective ones of the excitations transmitted by the previous AV-block scheme and transmitting the others with a predetermined transmittal delay, wherein the excitations transmitted by the last AV-block scheme constitute the calculated or simulated R-R-pattern.

In other words, the model is based on a multi-level block scheme consisting of at least two consecutive AV-blocks, but possibly three or more. Based on this model, a calculated or simulated R-R-pattern is obtained, which can be regarded as the prediction of what the R-R-pattern would be if the corresponding multi-level AV-block was actually present. The system further comprises consistency checking means adapted to automatically check whether said sequence of R-R-intervals of the ECG-data is to some predetermined degree consistent with the calculated or simulated R-R-pattern. If such consistency is found, this is a strong indication that the arrhythmic R-R-pattern is due to atrial flutter rather than atrial fibrillation. For this reason, the system is configured to identify the ECG-data to be based on atrial flutter if the predetermined degree of consistency is actually found.

This way, the system allows to identify atrial flutter which otherwise would not have been discerned from the ECG due to the aperiodic R-R-pattern generated by the multi-level AV-block reminiscent of "arrhythmia absoluta".

Preferably, the system is further configured to output information indicative of said consistency. This is particularly useful if the system operates in a watchdog-like manner. For example, if the system was integrated in an external or implanted ECG-processing device, it could continuously check whether the ECG-data is consistent with atrial flutter and a multi-level AV-block, and only if this type of consistency is found, it would output a corresponding message to the user.

Also, the predetermined degree of consistency is a somewhat soft threshold. A very precise consistency would give a high confidence level that a consistency actually exists, while a somewhat lesser degree of consistency may indicate that it is not so clear that the instant ECG R-R-pattern can actually be attributed to the AV-block scheme in question. In these cases, it is helpful to the cardiologist if the system also provides some sort of confidence value of the consistency.

In addition, the system is preferably adapted to output the hierarchy of AV-block schemes that led to the calculated R-R-pattern that was found to be consistent with said ECG R-R-pattern. This way, the system does not only allow to recognize atrial flutter underlying the arrhythmic R-R-pattern, but also informs the cardiologist about the multi-level block scheme responsible for this. Herein, the term "hierarchy of AV-block schemes" refers to the sequence of AV-block schemes applied one after another. That is to say, the first AV-block scheme applied to the atrial flutter signal would be the topmost AV-block scheme in the hierarchy, while the last AV-block scheme in sequence that actually yields the R-R-pattern would be the bottommost or lowest AV-black in the hierarchy.

In a preferred embodiment, one or more of the AV-block schemes may be a Wenckebach block scheme characterized by a cycle of progressive prolongations of the transmittal delay in consecutively transmitted excitation peaks, followed by a blocked peak, after which the original transmittal delay is resumed and the cycle is repeated. In the following, "excitation peaks" are referred to as "peaks" for short.

Herein, the Wenckebach block scheme may be characterized by
- an initial transmittal delay t_{cW} between receiving and transmitting the first peak after a peak has been blocked, and
- a transmittal delay increment Δt_{w} by which each transmittal delay in the cycle is increased over the transmittal delay of the previous peak until a transmittal delay threshold t_{rW} is exceeded, which leads to a block of the respective peak.

Note that this is only one way of parameterizing the characteristic Wenckebach block scheme. Clearly, more sophisticated models of the typical Wenckebach block scheme may likewise be employed, including those that may be formulated in the future based on an even better understanding of the physiological processes involved. The advantage of the above choice for parameterizing the Wenckebach block scheme, however, is that it can be completely defined with only three parameters, which allows for an easy and efficient mathematical handling of the model.

Preferably, one or more of the AV-block schemes may be a Mobitz block scheme characterized by blocking one or more peaks after each transmission. Herein, the Mobitz block scheme is preferably characterized by a fixed transmittal delay t_{cM} between receiving and transmitting a peak and by a refractory period t_{rM} after transmittal during which further received peaks, i.e. further excitations are blocked. Again, this is only one possible way of formulating the Mobitz block scheme, and more sophisticated formulations may be used instead. Nevertheless, it is believed that this model captures the essential physiological behaviour and leads to the correct conclusions.

Accordingly, the model used by the system of the invention may employ a multi-level AV-block consisting of two or more consecutive AV-blocks, where each of the AV-blocks may be either Wenckebach or Mobitz type.

The parameters are preferably chosen from the following physiologically meaningful ranges:
50 ms < t_{cM}< 700 ms, and/or
0 ms < t_{rM} < 800 ms, and/or
50 ms < t_{cW} < 700 ms, and/or
0 ms < Δt_{W} < 500 ms, and/or
0 ms < t_{rW} < 800 ms.

Further, the periodic or almost periodic signal representing excitation peaks of atrial flutter preferably has a frequency (or mean frequency) fₐ between 5.5 and 2.5 Hz.

In a preferred embodiment, the system is configured to systematically calculate R-R-patterns for a set of possible combinations of the AV-block scheme types (e.g. sequences of Wenckebach and Mobitz blocks) and AV-block scheme parameters (e.g. t_{cM}, t_{rM}, t_{cW}, Δt_{W}, t_{rW}) and to check, for each calculated R-R-pattern, for consistency with the R-R-pattern of the ECG-data. This can be regarded as a "forward simulation", in which an assumption is made about a possible multiblock scheme characterized by a sequence of different AV-block schemes and their respective characteristics defined by the AV-block scheme parameters, and where it is checked whether this assumption is consistent with the actual ECG-data. Due to the mathematical simplicity of the models described above, up to a number of three to four block levels, this can be easily done in a "brute force approach", where the entire parameter space of the multi-level block model is discretized and an R-R-pattern is calculated for the model according to each individual parameter set.

However, the invention is of course open for any type of improvements based on physiological considerations, correlations between parameters, a priori assumptions about parameters, and particularly more advanced mathematical and numerical methods to solve the "inverse simulation" problem. This will allow the treatment of even more block levels in reasonable computational time and application in real time. The inverse problem consists in determining parameters that maximize an objective function, in our case the statistical test value that yields the likelihood that the real and the simulated pattern coincide. Instead of trying and comparing all possible parameter combinations, advanced optimization algorithms can do this in a fraction of the computational time by logically excluding parameter ranges and making use of derivative information.

Preferably, the consistency checking means is adapted to determine the degree of consistency by calculating a total deviation of the excitation peak timings of the calculated and the ECG R-R-patterns based on statistical tests that yield a probability that the two patterns are identical. For example, one can calculate the sum of squares of deviations of corresponding excitation timings. Herein, a predetermined degree of consistency is found if the total deviation is less than a predetermined threshold value.

In case of uncertainty about the outcome of the consistency check, the presence of an assumed AV-block scheme can be verified by administering a pharmaceutical agent that affects one or both of the atrial excitation or the AV-block scheme of the patient in a predetermined way. For example, the pharmaceutical agent could be an agent that reduces the atrial excitation frequency. Alternatively, the pharmaceutical agent could be an agent that affects the transmittal delay between receiving and transmitting an excitation peak in an AV-block, or which changes the refractory period after transmittal of an excitation peak during which further excitations are blocked. This embodiment is based on the consideration that if a certain AV-block scheme is in fact present, the pharmaceutical agent will affect the ECG data in an expected way. Simply put, the system of the invention automatically checks whether the change in the ECG-data after administering the pharmaceutical agent is consistent with the assumed AV-block scheme, i.e. the AV-block scheme underlying the calculated or simulated R-R-pattern for which the consistency shall be confirmed. In cases where consistency is in question, this will give additional information based on which it can be decided with more certainty whether a given AV-block scheme is present or not.

In a preferred embodiment, the system is adapted to output information that a pharmaceutical agent should be administered. For example, if a system cannot give a definite answer about consistency, for example because some confidence value is too low, it may automatically suggest to further confirm consistency by administering such pharmaceutical agent. In this case, the system has some information as to how certain pharmaceutical agents will affect the atrial excitation or the AV-block scheme or both. In a preferred embodiment, the system is adapted to propose a certain pharmaceutical agent to the physician to administer, which in the present case may be the most suitable to confirm or disprove consistency.

The system of the invention may be formed by or included in an ECG-apparatus, in particular an implantable ECG-apparatus, such as a so-called event-recorder. However, the system may also be formed by or included in a pacer or a defibrillator. In yet a further embodiment, the system may be formed by a personal computer, a work station under suitable program control or as another diagnostic algorithm in an external ECG-apparatus.

The present invention also relates to a computer program product which when executed on a computer materializes a system according to one of the abovementioned embodiments. In particular, the computer program product may be a plug-in that may be added to existing software used in ECG-devices, event-recorders, pacers or the like, that generally adds one step of data analysis that the inventors believe is very important, namely to determine whether present ECG-data may be indicative of atrial flutter subjected to multi-level AV-blocks.

### BRIEF DESCRIPTION OF THE FIGURES

Fig. 1 is an ECG of a patient suffering atrial fibrillation.
Fig. 2 is a figure taken from Kosowsky et al. showing ECG-data attributed to a two-level AV-block.
Fig. 3 is a ladder diagram of a two-level AV-block scheme automatically discovered by the system of the invention based on the ECG-data of Fig. 2.
Fig. 4 is a ladder diagram of a two-level AV-block scheme automatically discovered by the system of the invention based on another ECG-data set taken from Kosowsky et al.

### DESCRIPTION OF THE PREFERRED EMBODIMENT

Fig. 2 is a figure taken from Kosowsky et al. showing ECG-data and a ladder diagram that Kosowsky et al. used to explain how the R-R-pattern of the ECG-data can be attributed to a periodic atrial signal subjected to a two-level AV-block, namely a Mobitz block followed by a Wenckebach block. In the publication of Kosowsky et al., the analysis has been made "by hand" to demonstrate that the physiological phenomenon of multi-level AV-block does exist.

When comparing the arrhythmic ECG of Fig. 2 with the ECG of Fig. 1, it is difficult to tell a qualitative difference. In fact, the ECG of Fig. 2, which is based on periodic P-waves subjected to a two-level AV-block looks very similar to the "arrhythmia absoluta" caused by atrial fibrillation. This is why the inventors conjecture that in many cases, atrial fibrillation has been erroneously diagnosed instead of atrial flutter "disguised" by multi-level AV-block.

The system of the invention allows to automatically distinguish atrial flutter from atrial fibrillation in case of multi-level AV-block. This holds especially true for cases of atrial flutter where the flutter waves are vanishingly small and do not further affirm the diagnosis. According to an embodiment of the invention, the system does this by assuming that a certain multi-level AV-block scheme is present, assuming atrial flutter with a given frequency and phase and calculating the R-R-pattern that will result if the atrial flutter is applied to the AV-node exhibiting the AV-block. If the calculated R-R-pattern is consistent with a given ECG-data set, this is an indication that atrial flutter, modified by multi-level AV-block, is actually present, rather than atrial fibrillation.

In an embodiment of the invention, all types of AV-block schemes that can reasonably be expected are systematically tested, and the resulting calculated R-R-patterns are checked for consistency with the ECG-data. Specifically, first two-level AV-blocks are tried, with all four possible combinations of Wenckebach-blocks and Mobitz blocks, and then higher level AV-blocks are tested.

In the model employed, each Wenckebach-block scheme is defined by an initial transmittal delay t_{cW} between receiving and transmitting the first peak after the peak has been blocked, and by a transmittal delay increment Δt_{w} by which each transmittal delay in the cycle is increased over the transmittal delay of the previous peak. If a transmittal delay threshold t_{rW} is exceeded, this leads to a block of the respective peak.

Also in the model employed, each Mobitz block scheme is defined by a fixed transmittal delay t_{cM} between receiving and transmitting a peak, and by a refractory period t_{rM} after transmittal during which further received peaks are blocked.

For each block scheme type (Wenckebach or Mobitz ), the parameter space of the respective parameters, i.e. t_{cW}, Δt_{w} and t_{rW} in case of Wenckebach and t_{cM} and t_{rM} in case of Mobitz , are varied within physiologically meaningful boundaries.

Each of the calculated R-R-patterns is compared with the ECG pattern for consistency. Specifically, the sum of squares of deviations of corresponding peak timings in the calculated R-R-pattern and the ECG R-R-pattern is calculated. If the sum of squares is below a threshold value, this is indicative of consistency. Needless to say, other, more sophisticated ways to give a quantitative account of the consistency may be employed. In this case, the system will identify the ECG data to be based on atrial flutter, and will output corresponding information to the user. In the preferred embodiment, the system will also output a confidence value of the consistency representing a probability that the diagnosis of atrial flutter in combination with a multi-level AV-block is correct.

Also, the system automatically or on demand outputs the details of the multi-level AV-block that has led to the consistent calculated R-R-pattern, i.e. the sequence and type of AV-blocks and the respective parameters characterizing the same.

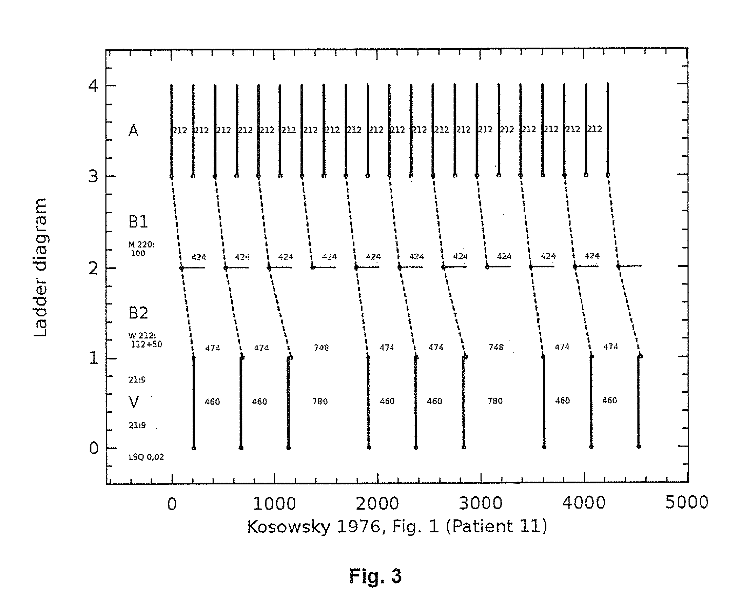

Fig. 3 is an example of a two-level AV-block scheme that has been found by the system of the invention to yield a R-R-pattern consistent with the ECG-data of Fig. 1 of Kosowsky et al., which is shown as Fig. 2 in the present application. As can be seen from Fig. 3, a good consistency was found by a two-level AV-block scheme consisting of
1.) a Mobitz block having a fixed transmittal delay t_{cM} of 100 ms and a refractor period t_{rM} of 220 ms, followed by
2.) a Wenckebach block characterized by an initial transmittal delay t_{cW} = 112 ms, a transmittal delay increment Δt_{w} of 50 ms and a transmittal delay threshold t_{rW} = 212 ms.

Comparing this with Fig. 2, it is seen that the analysis of the system of the invention is consistent with the finding of Kosowsky et al. for the same ECG-data, that was, however, carried out "by hand" and human intuition. However, note that the system of the invention has obtained this result automatically and based *only* on the R-R-pattern, no additional information of the ECG, such as P-wave timings has been employed. Instead, the frequency of the atrial flutter (a period of 212 ms) has actually been a result of the analysis provided by the system of the invention, not an input of the analysis.

Importantly, the system of the invention allows to automatically and routinely monitor ECG-data and to reliably and reproductively identify ECG-data that can be attributed to atrial flutter subjected to multi-level AV-block. The system can hence be employed as a "watchdog" that will alert the cardiologist of this situation when it arises. It is, however, the inventors' conjecture that this situation may arise much more often than one might presently think, since due to the similarity with atrial flutter R-R-patterns, and without the system of the invention, in clinical practice many instances will simply not be recognized and attributed to atrial fibrillation.

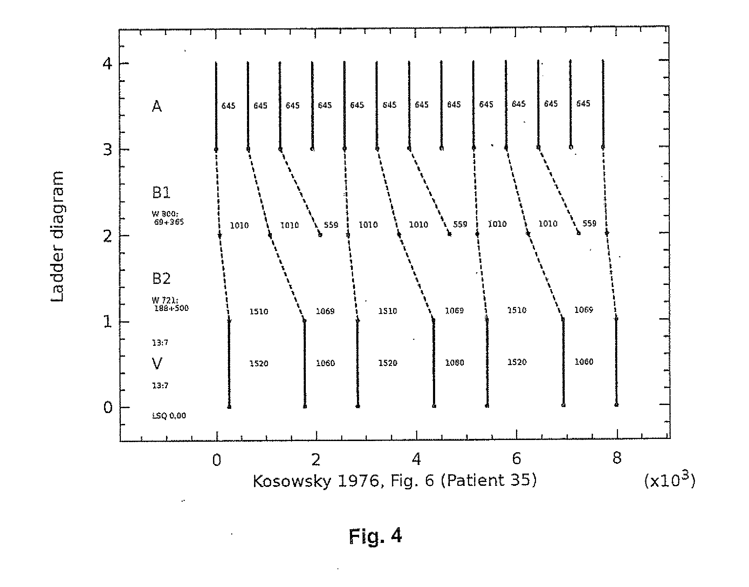

A further example demonstrating the system of the invention is based on the ECG-data of Fig. 6 of Kosowsky et al.. As shown in Fig. 4, again the system was able to trace back the ECG R-R-pattern of Fig. 6 of Kosowsky to a periodic P-wave with a two-level AV-block, where both block levels are of Wenckebach type.

As has been demonstrated by the above examples, the system of the invention indeed does allow to distinguish atrial flutter that is subjected to multi-level AV-block from atrial fibrillation. Accordingly, the invention provides technical means for helping the cardiologist to make a reliable diagnosis in this very situation which would appear to be difficult if not - at least with regard to higher level AV-blocks - impossible to make with the naked eye only. It is again emphasized that while the existence of multilevel AV-block per se might be known by experts in the field of electrophysiology e.g. from Kosowsky et al., to the knowledge of the inventors, it has never been considered by general cardiologists in actual clinical diagnosis. However, reliably distinguishing between atrial fibrillation and atrial flutter plus multilevel AV-block has become particularly important with regard to the PVI-method discussed above, where atrial flutter is a common complication, and where it is of highest importance that it is reliably distinguished from atrial fibrillation. Using the system of the invention, this can be very successfully achieved.

As mentioned before, the system of the invention may be formed by an ECG-apparatus, or may be included in an ECG-apparatus. A further useful embodiment of the invention is a computer program product, which generates or adds this functionality to an existing device, such as existing ECG-apparatuses.

Although the preferred exemplary embodiment is shown and specified in detail in the drawings and the preceding specification, this should be viewed as purely exemplary and not as limiting the invention. It is noted in this regard that only the preferred exemplary embodiment is shown and specified, and all variations and modifications should be protected that presently or in the future lay within the scope of protection of the invention.

## Claims

1. A system for automatically distinguishing atrial flutter from atrial fibrillation based on a sequence of R-R-intervals of ECG data, said system comprising means adapted to calculate or simulate an R-R-pattern from a model which is based on
- a periodic or almost periodic signal representing peaks of an atrial flutter signal,
- a first AV-block scheme blocking respective ones of the excitations corresponding to said peaks of the atrial flutter signal while transmitting the others with a predetermined transmittal delay,
- at least one more AV-block scheme blocking respective ones of the excitations transmitted by the previous AV-block scheme and transmitting the others with a predetermined transmittal delay, wherein the excitations transmitted by the last AV-block scheme constitute the calculated or simulated R-R-pattern,
the system further comprising consistency checking means adapted to automatically check whether said sequence of R-R-intervals of the ECG-data is to some predetermined degree consistent with said calculated or simulated R-R-pattern,
said system being configured to identify the ECG-data to be based on atrial flutter if said predetermined degree of consistency is found.

2. The system of claim 1, said system being further configured to output information indicative of said consistency, wherein said information indicative of said consistency preferably also includes a confidence value of the consistency, and/or
wherein the system is further adapted to output the hierarchy of AV-block schemes that led to the calculated R-R-pattern that was found to be consistent with said ECG R-R-pattern.

3. The system of one of the preceding claims, wherein one or more of the AV-block schemes is a Wenckebach block scheme **characterized by** a cycle of progressive prolongations of the transmittal delay in consecutively transmitted excitation peaks, followed by a blocked excitation peak, after which the original transmittal delay is resumed and the cycle is repeated.

4. The system of claim 3, wherein said Wenckebach block scheme is **characterized by**
- an initial transmittal delay t_{cW} between receiving and transmitting the first excitation peak after an excitation peak has been blocked,
- a transmittal delay increment Δt_{w} by which each transmittal delay in the cycle is increased over the transmittal delay of the previous excitation peak until a transmittal delay threshold t_{rW} is exceeded, which leads to a block of the respective excitation peak.

5. The system of one of the preceding claims, wherein one or more of the AV-block schemes is a Mobitz block scheme **characterized by** blocking one or more excitation peaks after each transmission.

6. The system of claim 5, wherein said Mobitz block scheme is **characterized by** a fixed transmittal delay t_{cM} between receiving and transmitting an excitation peak and by a refractory period t_{rM} after transmittal during which further received excitation peaks are blocked.

7. The system of claim 4 or 6, wherein the parameters are chosen from the following ranges:
50 ms < t_{cM} < 700 ms, and/or
0 ms < t_{rM} < 800 ms, and/or
50 ms < t_{cW} < 700 ms, and/or
0 ms < Δt_{w} < 500 ms, and/or
0 ms < t_{rW} < 800 ms.

8. The system of one of the preceding claims, wherein said periodic or almost periodic signal representing peaks of atrial flutter has a frequency or mean frequency fₐ between 5.5 and 2.5 Hz.

9. The system of one of the preceding claims, wherein said system is configured to systematically calculate R-R-patterns for each of a set of possible combinations of AV-block scheme types and AV-block scheme parameters, and to check, for each calculated R-R-pattern, for consistency with the R-R-pattern of the ECG data.

10. The system of one of the preceding claims, wherein the consistency checking means is adapted to determine the degree of consistency by calculating statistical tests of the two time series to be identical, in particular based on the sum of squares of deviations of corresponding excitation peak timings,
wherein a predetermined degree of consistency is found if said total deviation is less than a predetermined threshold value.

11. The system of one of the preceding claims, wherein the consistency checking means is further adapted to monitor a change in said sequence of R-R-intervals of ECG data after administering a pharmaceutical agent, wherein
said pharmaceutical agent affects one or both of
- atrial excitation, or
- an AV-block scheme, and in particular
● a transmittal delay between receiving and transmitting an excitation peak, or
● a refractory period after transmittal during which further excitation peaks are blocked,
in a predetermined manner, said consistency checking means being further adapted to check whether said change in said sequence of R-R-intervals of the ECG data is consistent with the AV-block scheme underlying the calculated or simulated R-R-pattern.

12. The system of claim 11, said system being further adapted to output information that such pharmaceutical agent, and preferably which pharmaceutical agent, should be administered.

13. The system of one of the preceding claims, said system being formed by or included in one of the following apparatuses:
- an external ECG apparatus,
- an implantable ECG apparatus,
- a pacer,
- a defibrillator, or
- a personal computer or work station under suitable program control.

14. A computer program product, which when executed on a computer causes the computer to carry out the following steps:
calculating an R-R-pattern from a model which is based on
- a periodic or almost periodic signal representing peaks of an atrial flutter signal,
- a first AV-block scheme blocking respective ones of the peaks of the atrial flutter signal while transmitting the others with a predetermined transmittal delay,
- at least one more AV-block scheme blocking respective ones of the peaks transmitted by the previous AV-block scheme and transmitting the others with a predetermined transmittal delay, wherein the peaks transmitted by the last AV-block scheme constitute the calculated R-R-pattern,
automatically checking whether said calculated R-R-pattern is to some predetermined degree consistent with a sequence of R-R-intervals of ECG data, and identifying the ECG data to be based on atrial flutter if said predetermined degree of consistency is found.

15. The computer program product of claim 14, which when loaded into the processor of a technical device materializes a system according to one of claims 1 to 12.
